# EUROPEAN PATENT APPLICATION

(11) **EP 2 548 554 A1**
(43) Date of publication of application: **23.01.2013**
(21) Application number: 11756368.4
(22) Date of filing: 16.03.2011
(51) Int. Cl.: A61K 31/19, A61P 21/00

(54) **INHIBITOR OF IN VIVO PROTEOLYSIS**

(30) Priority: 18.03.2010 JP 2010061755
(71) Applicant: The Nisshin OilliO Group, Ltd., Tokyo 104-8285 (JP)
(72) Inventor: SEKINE, Seiji, Yokosuka-shi Kanagawa 239-0832 (JP)
(74) Representative: Schrell, Andreas
(86) International application number: PCT/JP2011/056291
(87) International publication number: WO 2011/115184

(57) **Abstract**

An inhibitor of in vivo proteolysis, which comprises, as an active principle, a fatty acid-based compound containing one acyl group (RCO-) having 6 to 12 carbon atoms, preferably a fatty acid precursor which is converted into a fatty acid having 6 to 12 carbon atoms *in vivo.*

## Description

### Technical Field

The present invention relates to an inhibitor of *in* vivo proteolysis which contains, as an active principle, a fatty acid-based compound containing an acyl group having 6 to 12 carbon atoms.

### Background Art

*In vivo,* proteins are the center of the components of muscles, organs, skin, hairs, nails and the like. In these proteins, a cycle of protein synthesis and degradation is always repeated. When nutrient intake is adequate, the balance between the synthesis and degradation is kept. However, if protein degradation is enhanced from some causes including long-term and heavy exercises, long-term restriction to exercise, excessive diet, and the dietary reduction caused by aging, this causes reduction in muscular power particularly due to the atrophy or decline of a muscle, giving rise to various disorders *in vivo.*

In the case of sarcopenia as an example, muscular strength and muscular power are reduced with aging, which elicits a loss of muscle mass, exerting an adverse influence on daily life. In the case of disuse muscle atrophy, muscle atrophy progresses in a relative short time due to the restriction to exercises by long-term bed rest or fixation of a plaster cast or due to the depression of active mass of muscle by exposure to a gravity-free environment during a stay in space (Nair KS, Am J Clin Nutr, 81 (5), 953-963, 2005; and K. Goto and T. Ohira, Japanese Journal of Aerospace Environmental Medicine, 44, 49-58, 2007).

Protein energy malnutrition (PEM) is classified into a marasmus type and a kwashiorkor type. The marasmus type PEM is characterized by clear reduction in skeletal muscle or adipose tissue, showing significant reduction in body weight, whereas the kwashiorkor type PEM is characterized by significant reduction in visceral proteins due to inhibition of protein synthesis and hypercatabolism (M. Oga, Japanese Journal of Parenteral and Enteral Nutrition, 22, 439-445, 2007).

The both types are each known to be a reversible change leaving the possibility of complete recovery through nutrition therapy and exercise therapy. However, there is the case where the nutrition therapy or exercise therapy cannot be approprietly given to aged persons having reduced digestive/absorptive function and persons having reduced motion ability. Therefore, it is desired to develop medicaments inhibiting the enhancement of protein degradation in vivo. As such a medicament, only a few medicaments using food components have been developed so far, and, for example, a medicament containing lycopene as an active principle has been developed (Japanese Patent No. 4038088).

### Summary of Invention

### Problem to be solved by the Invention

The present invention has been made in view of the above circumstances and it is an object of the present invention to provide an inhibitor of *in vivo* proteolysis which has high safety and which is reduced in the fear of side effects even if administered continuously.

### Means for solving the Problem

The present inventors have made earnest studies to solve the above problem. As a result, they have found that the above problem can be solved by inhibiting the expression of an enzyme participating in *in vivo* proteolysis (hereinafter referred to as a proteolysis factor) by the action of a fatty acid having 6 to 12 carbon atoms (hereinafter referred to as a medium-chain fatty acid) as an active principle, thereby completing the present invention.

The medium-chain fatty acid is an oil and fat component consumed by humans according to their dietary habits, that is, an oil and fat component contained in edible oils and foods such as dairy products. The medium-chain fatty acid is usually ingested in the form of acylglycerol where the medium-chain fatty acid is ester-bonded with glycerin. It is known that when the acylglycerol having the medium-chain fatty acid is ingested by eating, it is rapidly degraded and absorbed into a medium-chain fatty acid in the digestive tract, and is rapidly converted into energy in the liver. From such characteristics, the medium-chain fatty acid is used as an energy source having high safety for the purpose of supplying energy to persons having reduced digestive/absorptive function or recovering the physical strength after operations in medical treatments. However, there has been no report concerning the effect of the medium-chain fatty acid on inhibition of *in vivo* proteolysis.

According to a first aspect of the present invention, there is provided an inhibitor of *in vivo* proteolysis as explained below.
(1) An inhibitor of in vivo proteolysis, which comprises, as an active principle, a fatty acid-based compound containing one acyl group (RCO-) having 6 to 12 carbon atoms.
(2) The inhibitor of *in* vivo proteolysis according to (1), wherein the fatty acid-based compound is
   a fatty acid having 6 to 12 carbon atoms, or
   a fatty acid precursor which is converted into a fatty acid having 6 to 12 carbon atoms *in vivo.*
(3) The inhibitor of in vivo proteolysis according to (2), wherein the fatty acid is a fatty acid having 8 carbon atoms, a fatty acid having 10 carbon atoms, or a mixture of a fatty acid having 8 carbon atoms and a fatty acid having 10 carbon atoms.
(4) The inhibitor of *in* vivo proteolysis according to any one of (1) to (3), wherein the fatty acid-based compound is an ester of a fatty acid and glycerin.
(5) The inhibitor of in vivo proteolysis according to any one of (1) to (4), wherein the protein is a myoprotein.

According to another aspect of the present invention, there is provided a pharmaceutical composition for preventing or treating a disease caused by the enhancement of *in vivo* proteolysis, which comprises a fatty acid-based compound containing one acyl group having 6 to 12 carbon atoms and a pharmaceutically acceptable excipient.

According to a further aspect of the present invention, there is provided a method for preventing or treating a disease caused by the enhancement of *in vivo* proteolysis, which comprises administering an effective amount of a fatty acid-based compound containing one acyl group having 6 to 12 carbon atoms to a subject in need thereof.

According to a further aspect of the present invention, there is provided a use of a fatty acid-based compound containing one acyl group having 6 to 12 carbon atoms for the preparation of a medicament for preventing or treating a disease caused by the enhancement of *in* vivo proteolysis.

### Effects of Invention

The proteolysis inhibitor according to the present invention can be used to prevent or treat a disease caused by the enhancement of *in vivo* proteolysis, and can be particularly used to prevent or treat a disease, such as sarcopenia and disuse muscle atrophy, caused by the enhancement of a myoprotein degradation. Here, the prevention means, for example, the suppression or delay of onset of a disease, or the like, and the treatment means the delay of progress, alleviation, relief, improvement, or healing of symptoms, or the like. Further, the proteolysis inhibitor according to the present invention contains, as an active principle, a fat and oil component consumed by humans according to their dietary habits, and is therefore highly safe, so that the fear of side effects is lessened. Thus, the proteolysis inhibitor according to the present invention can be ingested without anxiety.

### Mode for carrying out the Invention

Embodiments of the present invention will be explained in detail. The present invention is not limited to the following embodiments at all, and may be properly modified to practice within the object of the present invention.

In the inhibitor of *in* vivo proteolysis according to the present invention, a fatty acid having 6 to 12 carbon atoms (hereinafter referred to as a medium-chain fatty acid), which is an active principle, itself acts in a tissue where the protein degradation is enhanced. Accordingly, the inhibitor of in vivo proteolysis is only required to exist in the form of a medium-chain fatty acid when it acts *in vivo.* When the inhibitor is administered, any form may be used without any particular limitation, insofar as it is a fatty acid-based compound containing one acyl group having 6 to 12 carbon atoms. The inhibitor may have any of the form of a medium-chain fatty acid and the form of a fatty acid precursor (for example, a salt or an ester) which is to be converted into a medium-chain fatty acid in vivo. The inhibitor preferably has the form of acylglycerol from the viewpoint of safety based on conventional dietary experiences.

Specifically, the fatty acid-based compound in the present invention includes a fatty acid, fatty acid salt, and fatty acid ester.

In the present invention, the medium-chain fatty acid may be obtained, for example, by hydrolyzing palm kernel oil or coconut oil, and then refining it. Further, a commercially available product or a reagent may be used. The medium-chain fatty acid may be constituted of one type of medium-chain fatty acid or a plurality of types of medium-chain fatty acids. The medium-chain fatty acid is preferably a saturated fatty acid having an even number of carbon atoms; more preferably, a fatty acid having 8 carbon atoms, a fatty acid having 10 carbon atoms, or a mixture of a fatty acid having 8 carbon atoms and a fatty acid having 10 carbon atoms; and even more preferably a saturated fatty acid having 8 carbon atoms.

The above acylglycerol is obtained by combining a medium-chain fatty acid with glycerin by ester-bonding. The acylglycerol may have the forms of monoacylglycerol, diacylglycerol, and triacylglycerol, depending on a difference in the degree of esterification. Further, the diacylglycerol and triacylglycerol may be constituted of the same types of medium-chain fatty acids or different types of medium-chain fatty acids. In the case where the acylglycerol is constituted of different types of medium-chain fatty acids, no particular limitation is imposed on the bonding position of each medium-chain fatty acid to glycerin.

Although the acylglycerol may be used either singly or in combinations of two types or more, the form of triacylglycerol is desirable from the viewpoint of medium-chain fatty acid intake efficiency and dietary experiences.

Although no particular limitation is imposed on a method of preparing an acylglycerol constituted of medium-chain fatty acid, it may be obtained, for example, by an esterification reaction between a medium-chain fatty acid derived from palm kernel oil or coconut oil and glycerin. Examples of methods of the esterification reaction include a method of reacting them under pressure without using catalyst or solvent, a method of reacting them using a synthetic catalyst such as sodium methoxide, and a method of reacting them using lipase as a catalyst. Further, a triglyceride which is generally called MCT (medium-chain triglycerides) and is constituted of a saturated medium-chain fatty acid having 8 to 10 carbon atoms such as coconut oil-degraded fatty acid is preferably used.

Examples of a method for investigating the content of the medium-chain fatty acid in the inhibitor of in vivo proteolysis of the present invention include a method of methyl-esterifying the medium-chain fatty acid in the inhibitor of *in* vivo proteolysis and analyzing it by gas chromatography.

The inhibitor of *in vivo* proteolysis according to the present invention can be used to inhibit the degradation of a protein existing in any tissue in vivo and, particularly, to inhibit the degradation of a myoprotein which is a protein existing in muscles. The inhibitor of *in vivo* proteolysis according to the present invention shows the effect of preventing or treating a disease caused by the enhancement of in vivo proteolysis. Examples of the disease caused by the enhancement of *in* vivo proteolysis include sarcopenia, disuse muscle atrophy, protein energy malnutrition (PEM), rhabdomyolysis, muscular dystrophy, and increase in urea nitrogen and creatinine caused by renal failure. The medium-chain fatty acid used in the present invention widely exists in nature and is contained in edible natural products, and thus it has high safety. Therefore, the proteolysis inhibitor according to the present invention can be safely used in medical applications.

When the inhibitor of *in* vivo proteolysis according to the present invention is used in medical applications, either oral administration or parenteral administration can be randomly selected as the administration method. Examples of the dosage form for oral administration include preparations such as a capsule, tablet, pill, powder, fine granule, granule, solution and syrup. Examples of the dosage form for parenteral administration include preparations such as a injection and infusion. Moreover, these preparations are preferably made into the form of a composition containing the medium-chain fatty acid or its precursor, which is an active principle, and a pharmacologically and pharmaceutically acceptable additive. As the additive, a substance is generally used which is commonly used as an excipient in pharmaceutical fields and do not react with the active principle used in the present invention.

When the inhibitor of in vivo proteolysis according to the present invention is combined with an already available drug, this can reduce the dosage of the already available drug and hence the side effects caused by the drug. As for the combination with another drug, the proteolysis inhibitor and the other drug may be included in the same pharmeceutical composition like a combined drug, or in a different pharamaceutical composition separately.

The dosage of the proteolysis inhibitor according to the present invention may be properly determined depending on various conditions such as the symptom, either prevention or treatment, age, and weight of a patient, administrating method, and administrating period. The dosage of the proteolysis inhibitor according to the present invention may be, for example, 0.01 to 1 g/kg weight/day in terms of the amount of the medium-chain fatty acid.

A subject to which the proteolysis inhibitor according to the present invention is administered is any animal, preferably a mammal, and more preferably humans.

### Examples

The present invention will be explained in more detail by way of Examples, but the present invention is not limited by the Examples.

In the Examples, the present inventors investigated the effect of a medium-chain fatty acid and a long-chain fatty acid, which are ingested in the form of fats and oils in usual diets, on the inhibition of *in* vivo proteolysis.

In the degradation of proteins in vivo, there are three primary pathways, that is, a ubiquitin-proteasome system, calpain-calpastatin system, and lysosome system. Among these pathways, the present inventors focused attention on the ubiquitin-proteasome system and calpain-calpastatin system in the Examples.

The ubiquitin-proteasome system is a system in which a protein is modified with ubiquitin by the action of ubiquitin ligase and it is degraded by the action of proteasome, wherein the rate of the degradation is determined by the step of ubiquitin modification by the action of ubiquitin ligase. On the other hand, the calpain-calpastatin system is a system in which various degradation enzymes are activated by the action of calpain and the action of calpain is controlled by calpastatin.

Therefore, it is considered that *in vivo* proteolysis can be inhibited if the expression level of ubiquitin and ubiquitin ligase can be inhibited in the ubiquitin-proteasome system, or if the expression level of calpain can be inhibited in the calpain-calpastatin system.

Further, with regard to the degradation of a skeletal muscle protein in the ubiquitin-proteasome system, it is reported that the degradation of a skeletal muscle protein is enhanced in a septic rat, as ubiquitin mRNA is increased in the skeletal muscle of the rat (Reference: Tiao G, Fagan JM, Samuels N, James JH, Lieberman M, Fischer JE, Hasselgren PO, J Clin Invest, 94 (6), 2255-2264, 1994). Therefore, the expression level of ubiquitin mRNA is a useful index of protein degradation in muscle.

Therefore, in the Examples, the present inventors investigated the effect on the inhibition of proteolysis by measuring each expression level of ubiquitin (ubiquitin b [Ubb]) and two types of ubiquitin ligase mRNAs (muscle atrophy F-box protein [MAFbx] and muscle ring finger protein 1 [MuRF]), as well as the expression level of two types of calpain mRNAs (calpain 1 [Capn1] and calpain 2 [Capn2]).

### [Test Example 1] Investigation of the effect on the inhibition of proteolysis (1)

In order to investigate the effect on the inhibition of proteolysis *in vitro,* the expression level of mRNA was measured in cultured cells to which various fatty acids were added.

### (Cultured cells)

As the cultured cells, myoblast cell lines L6 derived from a rat skeletal muscle supplied from European Collection of Cell Cultures were used.

### (Normal medium)

A normal medium was prepared by adding 10% fetal bovine serum (MP manufactured by Biomedical, Inc.) to DEM medium (manufactured by Sigma-Aldrich Inc.) and further adding 100 unit/mL of penicillin and 100 µg/mL of streptomycin, which were antibiotics, to the medium.

### (Differentiation-induction culture medium)

A differentiation-induction culture medium was prepared by adding 2% fetal bovine serum (MP manufactured by Biomedical, Inc.) to DEM medium (manufactured by Sigma-Aldrich Inc.) and further adding 100 unit/mL of penicillin and 100 µg/mL of streptomycin to the medium.

### (Preparation of test cells)

As the test cells, myotube cells were prepared as follows: L6 cells were inoculated to 10 mL of the normal medium in a 9.2 cm² Petri dish (manufactured by Techno Plastic Products Limited), and cultured in the medium under the condition of 5% carbon dioxide and 95% air at 37°C. After the cells came to a confluent state (that is, a dense state in the entire culture dish), they were cultured in the differentiation-induction culture medium under the condition of 5% carbon dioxide and 95% air at 37°C for 2 days to induce differentiation. Thereby, myotube cells were obtained.

### (Proteolysis inhibitor and Comparative Product)

As proteolysis inhibitors, n-octanoic acid (Example 1, trade name: n-Octanoic acid, manufactured by Sigma-Aldrich Inc.) which was a saturated fatty acid having 8 carbon atoms and n-decanoic acid (Example 2, trade name: n-Decanoic acid, manufactured by Sigma-Aldrich Inc.) which was a saturated fatty acid having 10 carbon atoms were used. As a comparative product, stearic acid (Comparative Example 1, manufactured by Wako Pure Chemical Industries, Ltd.) which was a saturated fatty acid having 18 carbon atoms was used.

The proteolysis inhibitor and the comparative product were each used in the form of a 50 mM solution in DMSO (manufactured by Sigma-Aldrich Inc.) to make it easy to add it to the test cell-containing culture solution.

### (Preparation of a test sample)

Each of the proteolysis inhibitors and comparative product was added at the concentration of 50 µmol/L in the test cell-containing culture solution. The obtained solution was cultured under the condition of 5% carbon dioxide and 95% air at 37°C for one day to prepare a test sample. Further, blank was prepared by adding only DMSO to the test cell-containing culture solution and culturing it under the same condition. Five samples were prepared for each of the proteolysis inhibitor, comparative product, and blank.

### (Method of measuring the amount of mRNA)

mRNA in the test sample was extracted using an RNeasy Mini Kit (manufactured by Qiagen Gmbh). Each of the extracted RNA having an amount corresponding to 500 ng was reverse-transcribed to cDNA by using a Transcriptor First Strand cDNA Synthetic Kit (manufactured by Roche Diagnostics K.K.).

Next, 1 µL of obtained cDNA was quantitatively measured by a real-time RT-PCR method using the primer pair shown below and utilizing a LightCycler quick system 330 (manufactured by Roche Diagnostics K.K.) in 20 µL system. Reaction protocol is as follows: thermal denaturation at 94°C for 30 seconds, annealing at 55°C for 30 seconds, and elongation reaction at 72°C for one minute (30 cycles). A Platinum Quantitave PCR SuperMix-UDG (manufactured by Invirtogen Corporation) was used as a PCR reaction reagent.

Further, ribosomal protein S29 (RPS29) mRNA, which was a house-keeping gene, was quantitatively measured as a control for correcting the variations of the measured values in each test sample. Then, the measured value of each test sample was divided by the measured value of RPS29 mRNA to calculate a relative value.

### (Primer)

### (Ubiquitin mRNA)

Ubb (Genbank accession number NM138895)

Ubb-Forward Primer:
5'-GAGTCAACCCTGCACCTGGTC-3' (SEQ ID No: 1)

Ubb-Reverse Primer:
5'-AGTAATGCCATCAGTGCCCG-3' (SEQ ID No: 2)

### (Ubiquitin ligase mRNA)

MAFbx (Genbank accession number AY059628)

MAFbx-Forward Primer:
5'-TCAAAGGTCTCACGATCACCG-3' (SEQ ID No: 3)

MAFbx-Reverse Primer:
5'-TCAGCCTCTGCATGATGTTCAG-3' (SEQ ID No: 4)

MuRF (Genbank accession number AY059627)

MuRF-Forward Primer:
5'-ACATCTTCCAGGCTGCCAATC-3' (SEQ ID No: 5)

MuRF-Reverse Primer:
5'-GACCTCCAGACATGGACACCG-3' (SEQ ID No: 6)

### (Calpain mRNA)

Capn1 (Genbank accession number NM019152)

Capn1-Forward Primer:
5'-CATAAGCACTCATGCTGCCAGA-3' (SEQ ID No: 7)

Capn1-Reverse Primer:
5'-CGAAATTACCTGACCATCTTCCG-3' (SEQ ID No: 8)

Capn2 (Genbank accession number NM017116)

Capn2-Forward Primer:
5'-GGAGCTAACAGGGCAGACCAAC-3' (SEQ ID No: 9)

Capn2-Reverse Primer:
5'-GAGGTTGATGAAGGTATCTGACCG-3' (SEQ ID No: 10)

### (Control mRNA)

RPS29 (Genbank accession number NM012876)

RPS29-Forward Primer:
5'-AAGATGGGTCACCAGCAGCTCTACT-3' (SEQ ID No: 11)

RPS29-Reverse Primer:
5'-AGACGCGGCAAGAGCGAGAA-3' (SEQ ID No: 12)

| Table 1 Expression level of mRNA (×10⁻³) | | |
|---|---|---|
| mRNA of proteolysis factor | Example 1 (Addition of n-octanoic acid) | Example 2 (Addition of n-decanoic acid) |
| Ubb | 12.1±0.6 *** | 14.7±1.1 * |
| MAFbx | 2.6±0.1 ** | 3.4±0.3 |
| MuRF | 1.3±0.1 ** | 1.6±0.1 |
| Capn1 | 0.3±0.0 *** | 0.3±0.0 |
| Capn2 | 631.6±49.9 *** | 738.1±90.7 |

| | | |
|---|---|---|
| Average value of 5 samples ± Standard error * A statistically significant difference from Comparative Example 1 is observed (p < 0.05) ** A statistically significant difference from Comparative Example 1 is observed (p < 0.01) *** A statistically significant difference from Comparative Example 1 is observed (p < 0.001) The Student's t-test was used for the significant difference test and it was determined that there was a significant difference when the significance level was less than 5%. | | |

| Table 2 Expression level of mRNA (×10⁻³) | | |
|---|---|---|
| mRNA of proteolysis factor | Comparative Example 1 (Addition of stearic acid) | Blank (Addition of DMSO) |
| Ubb | 18.2±0.8 | 18.3±1.7 |
| MAFbx | 4.2±0.2 | 3.6±0.5 |
| MuRF | 2.0±0.1 | 1.8±0.2 |
| Capn1 | 0.4±0.0 | 0.4±0.1 |
| Capn2 | 903.4±68.1 | 876.0±96.3 |

| | | |
|---|---|---|
| Average value of 5 samples ± Standard error | | |

From the results shown in Tables 1 and 2, Example 1 (addition of n-octanoic acid having 8 carbon atoms) and Example 2 (addition of n-decanoic acid having 10 carbon atoms) exhibited a lower expression level of proteolysis factor mRNA than Comparative Example 1 (addition of stearic acid having 18 carbon atoms) in all the cases of Ubb, MAFbx, MuRF, Capn1, and Capn2. Particularly, Example 1 exhibited a significantly lower expression level. The Student's t-test showed that n-octanoic acid could significantly inhibit the expression level of proteolysis factor mRNA. n-Decanoic acid showed a significant expression inhibiting effect on Ubb and also lowered the expression level of other proteolysis factor mRNA without increasing the expression level. Therefore, the results of the experiment showed that n-decanoic acid could inhibit the degradation of a protein in a cell. Further, Comparative Example 1 exhibited a similar level to the blank.

### [Test Example 2] Investigation of the effect on the inhibition of proteolysis (2)

In order to investigate the effect on the inhibition of proteolysis *in vivo,* the expression level of mRNA was measured in a muscle which was collected from a rat after the rat was made to ingest various fatty acids in the form of triacylglycerol. Further, the content of a medium-chain fatty acid in plasma was measured.

### (Experimental animal)

As the experimental animal, Wister male rats (three weeks of age) (purchased from Japan SLC Inc.) were used.

### (Test food)

The test food was designed to be a composition in which the content of milk casein as a protein source was decreased to 25% of the "ordinary food (AIN-93G)" in order to destroy the balance between protein synthesis and degradation in a rat, thereby enhancing the degradation of a protein. Specifically, the test food was designed to be each composition of "low-protein vegetable oil diet" and "low-protein MCT diet" as shown in Table 3.

With regard to the preparation of the test feed, first, a powder of each component other than oils and fats (MCT and soybean oil) was mixed, and finally, the oils and fats were blended and homogenized to make an "ordinary food", "low-protein vegetable oil food" and "low-protein MCT food. 1% by mass of soybean oil was added in the "low-protein MCT food" for the purpose of supplying essential fatty acids.

| Table 3 Composition of test food (mass%) | | | |
|---|---|---|---|
| Component | Ordinary food | Low-protein Vegetable oil food | Low-protein MCT food |
| Corn starch | 39.7486 | 54.7486 | 54.7486 |
| Milk casein | 20 | 5 | 5 |
| Gelatinized corn starch | 13.2 | 13.2 | 13.2 |
| Granulated sugar | 10 | 10 | 10 |
| Soybean oil 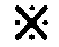 ¹ | 7 | 7 | 1 |
| MCT 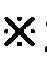 ² | 0 | 0 | 6 |
| Cellulose powder | 5 | 5 | 5 |
| Mineral mix | 3.5 | 3.5 | 3.5 |
| Vitamin mix | 1 | 1 | 1 |
| L-cystine | 0.3 | 0.3 | 0.3 |
| Choline bitartrate | 0.25 | 0.25 | 0.25 |
| Tertiary butyl hydroquinone | 0.0014 | 0.0014 | 0.0014 |

| | | | |
|---|---|---|---|
| 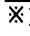 1 Soybean oil: trade name: "Nisshin Soybean Refined Oil", manufactured by The Nisshin OilliO Group, Ltd. 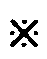 2 MCT [medium-chain fatty acid triglyceride]: trade name: "ODO", manufactured by The Nisshin OilliO Group, Ltd. | | | |

The soybean oil includes palmitic acid, stearic acid, oleic acid, linoleic acid, and α-linolenic acid as its major components. MCT (medium-chain fatty acid triglyceride) is a triglyceride obtained by combining n-octanoic acid (medium-chain fatty acid) and n-decanoic acid (medium-chain fatty acid) with glycerin by ester bonding.

### (Rearing method)

The test was made in three groups represented by a "low-protein MCT food ingestion group (Example 2)" ingesting a "low-protein MCT food", a "low-protein vegetable oil food ingestion group (Comparative Example 2) ingesting a "low-protein vegetable oil food", and a "control group (Control Example 1) ingesting an "ordinary food". Each group consists of 9 rats.

In the rearing method, each rat was individually reared in a stainless-steel mesh cage in an environment of 23 ± 1°C temperature and 50 ± 10% humidity for 12 hours in light-dark cycles (8:00 to 20:00 illumination). After each rat had been reared and acclimated for 2 days, ingestion of the test food (Table 3) was effected to rear the rats for 15 days. Further, restricted feeding treatment was performed so that the amounts of ingestion in the "low-protein MCT food ingestion group (Example 2)" and "low-protein vegetable oil food ingestion group (Comparative Example 2)" were respectively 60% by mass of that of the control group (Control Example 1).

### (Method of collecting a test sample)

In the process of collecting a test sample, the rat was anesthetized with ether to vivisect three hours after the last food intake on the last day of rearing. The right hind leg muscle of the rat was collected, and then blood samples were collected from the heart.

### (Method of measuring the amount of mRNA)

The quantitative measurement of mRNA was carried out in the same manner as in Test Example 1, except that mRNA was extracted from the right hind leg muscle of the rat using an RNeasy Fibrous Tissue Mini Kit (manufactured by Qiagen Gmbh).

| Table 4 Expression level of mRNA (×10⁻²) | | |
|---|---|---|
| mRNA of proteolysis factor | Example 2 (Low-protein MCT food ingestion group) | Comparative Example 2 (Low-protein vegetable oil food ingestion group) |
| Ubb | 7.9±0.2 * | 8.7±0.2 |
| MAFbx | 1.5±0.1 | 1.5±0.0 |
| MuRF | 3.9±0.2 | 4.2±0.1 |
| Capn1 | 7.2±0.2* | 8.6±0.4 |
| Capn2 | 449.0±16.1 | 436.0±10.1 |

| | | |
|---|---|---|
| Average value of 9 samples ± Standard error * A statistically significant difference from Comparative Example 2 is observed (p < 0.05) The Student's t-test was used for the significant difference test and it was determined that there was a significant difference when the significance level was less than 5%. | | |

| Table 5 Expression level of mRNA (×10⁻²) | |
|---|---|
| mRNA of Proteolysis Factor | Control Example 1 (Control group: ordinary food ingestion group) |
| Ubb | 10.3±0.3 |
| MAFbx | 3.5±0.2 |
| MuRF | 6.0±0.2 |
| Capn1 | 11.0±0.2 |
| Capn2 | 573.4±12.1 |

| | |
|---|---|
| Average value of 9 samples ± Standard error | |

From the results of Tables 4 and 5, the Example exhibited a lower expression level of proteolysis factor mRNA than the Comparative Example in the case of Ubb, MuRF, and Capn1. With regard to MAFbx and Capn2, the Example showed almost the same expression level of mRNA as the Comparative Example. With regard to Ubb and Capn1, the effect of inhibiting the expression level of proteolysis factor mRNA was clearly proved by the Student's t-test. Therefore, the results of the experiment showed that medium-chain fatty acids could inhibit the degradation of a protein in a rat.

In addition to the Example, the Comparative Example exhibited a lower expression level of each mRNA than the Control Example (untreated). This reason was considered to be that the rat was kept in the condition of malnutrition for a long period, so that the proteolysis *in vivo* was enhanced, bringing about the reduction of a protein of a substrate, which was also accompanied by the reduction of a protein degradation enzyme.

### (Method of measuring the content of medium-chain fatty acids in plasma)

The content of medium-chain fatty acids in plasma was measured as follows: Collected blood was transferred to a centrifugal tube filled with heparin sodium and subjected to centrifugation at 3,000 rpm for 10 minutes to obtain a supernatant as plasma. The content of medium-chain fatty acids in the plasma was measured according to the "Standard oil and fat test method (1996)".

| Table 6 Content of medium-chain fatty acids in the rat plasma (µg/mL) | | | |
|---|---|---|---|
| Fatty acid composition | Example 2 (Low-protein MCT food ingestion group) | Comparative Example 2 (Low-protein vegetable oil food ingestion group) | Control Example 1 (Control group: ordinary food ingestion group) |
| C8:0 | 23.7±1.1 | ND | ND |
| C10:0 | 40.3±1.5 | ND | ND |

From the results of Table 6, it was seen that the content of medium-chain fatty acids in the rat plasma was as follows: 23.7 µg/mL (164 µM) of n-octanoic acid (C8:0) and 40.3 µg/mL (234 µM) of n-decanoic acid (C10:0) was detected in Example 2, and no medium-chain fatty acid was detected in Comparative Example 2. It is considered that if a food containing MCT, triacylglycerol having medium-chain fatty acids, is ingested, it is absorbed and metabolized, and then supplied in the form of medium-chain fatty acids to muscle from blood to inhibit the degradation of a protein in the muscle.

It was clarified from the results of the above in-vitro and in-vivo tests that n-octanoic acid and n-decanoic acid, which are medium-chain fatty acids, could inhibit the expression of mRNA of a proteolysis factor. It is considered from the results that the medium-chain fatty acids could inhibit the pathways of degradation of proteins of the ubiquitin-proteasome system and calpain-calpastatin system and hence, the degradation of proteins *in vivo.*

## Claims

1. An inhibitor of *in* vivo proteolysis, which comprises, as an active principle, a fatty acid-based compound containing one acyl group (RCO-) having 6 to 12 carbon atoms.

2. The inhibitor of *in vivo* proteolysis according to claim 1, wherein the fatty acid-based compound is
a fatty acid having 6 to 12 carbon atoms, or
a fatty acid precursor which is converted into a fatty acid having 6 to 12 carbon atoms *in vivo.*

3. The inhibitor of *in* vivo proteolysis according to claim 2, wherein the fatty acid is a fatty acid having 8 carbon atoms, a fatty acid having 10 carbon atoms, or a mixture of a fatty acid having 8 carbon atoms and a fatty acid having 10 carbon atoms.

4. The inhibitor of *in vivo* proteolysis according to any one of claims 1 to 3, wherein the fatty acid-based compound is an ester of a fatty acid and glycerin.

5. The inhibitor of *in* vivo proteolysis according to any one of claims 1 to 4, wherein the protein is a myoprotein.


SEQUENCE LISTING

<110> The Nisshin OilliO Group, Ltd.

<120> An agent for inhibiting degradation of in vivo protein

<130> 11S0281P

<150> JP 2010-061755
<151> 2010-03-18

<160> 12

<170> PatentIn version 3.5

<210> 1
<211> 21
<212> DNA
<213> Artificial Sequence

<220>
<223> Forward primer for Ubb gene

<400> 1
gagtcaaccc tgcacctggt c 21

<210> 2
<211> 20
<212> DNA
<213> Artificial Sequence

<220>
<223> Reverse primer for Ubb gene

<400> 2
agtaatgcca tcagtgcccg 20

<210> 3
<211> 21
<212> DNA
<213> Artificial Sequence

<220>

<223>  Forward primer for MAFbx gene

<400>  3
tcaaaggtct cacgatcacc g                                                21


<210>  4
<211>  22
<212>  DNA
<213>  Artificial Sequence

<220>
<223>  Reverse primer for MAFbx gene

<400>  4
tcagcctctg catgatgttc ag                                               22


<210>  5
<211>  21
<212>  DNA
<213>  Artificial Sequence

<220>
<223>  Forwad primer for MuRF gene

<400>  5
acatcttcca ggctgccaat c                                                21


<210>  6
<211>  21
<212>  DNA
<213>  Artificial Sequence

<220>
<223>  Reverse primer for MuRF gene

<400>  6
gacctccaga catggacacc g                                                21


<210>  7
<211>  22
<212>  DNA

<213>  Artificial Sequence

<220>
<223>  Forward primer for Capn1 gene

<400>  7
cataagcact catgctgcca ga                                                22


<210>  8
<211>  23
<212>  DNA
<213>  Artificial Sequence

<220>
<223>  Reverse primer for Capn1 gene

<400>  8
cgaaattacc tgaccatctt ccg                                               23


<210>  9
<211>  22
<212>  DNA
<213>  Artificial Sequence

<220>
<223>  Forward primer for Capn2 gene

<400>  9
ggagctaaca gggcagacca ac                                                22


<210>  10
<211>  24
<212>  DNA
<213>  Artificial Sequence

<220>
<223>  Reverse primer for Capn2 gene

<400>  10
gaggttgatg aaggtatctg accg                                              24

<210> 11
<211> 25
<212> DNA
<213> Artificial Sequence

<220>
<223> Forward primer for RPS29 gene

<400> 11
aagatgggtc accagcagct ctact 25

<210> 12
<211> 20
<212> DNA
<213> Artificial Sequence

<220>
<223> Reverse primer for RPS29 gene

<400> 12
agacgcggca agagcgagaa 20